# EUROPEAN PATENT SPECIFICATION

(11) **EP 1 648 375 B1**
(45) Date of publication and mention of the grant of the patent: **12.11.2008**
(21) Application number: 04741340.6
(22) Date of filing: 30.07.2004
(51) Int. Cl.: A61K 6/00, A61K 6/087

(54) **DENTAL ROOT CANAL SEALING COMPOSITION**
ZUSAMMENSETZUNG ZUR ABDICHTUNG VON ZAHNWURZELKANÄLEN
COMPOSITION POUR RENDRE ETANCHE UN CANAL RADICULAIRE

(30) Priority: 31.07.2003 EP 03017390; 10.03.2004 US 551347 P
(43) Date of publication of application: 26.04.2006
(73) Proprietor: DENTSPLY DETREY GmbH, 78467 Konstanz (DE)
(72) Inventor: KLEE, Joachim, E., 78315 Radolfzell (DE)
(74) Representative: Hartz, Nikolai
(86) International application number: PCT/EP2004/008598
(87) International publication number: WO 2005/013921

(56) References cited:
- EP-A- 0 673 637
- WO-A-02/13767
- US-A1- 2003 045 604

## Description

### Field of the invention

The present invention relates to a dental root canal sealing composition curable by addition polymerisation in the absence of a polymerisation catalyst. The dental root canal sealing composition of the invention is provided in the form of a two-component composition.

### Background art

Dental root canal sealing compositions are frequently applied into the root canal through a canal of a needle. Due to the small dimensions of the needle canal, the compositions are required to have a low viscosity. Alternatively, dental root canal sealing compositions are applied by using lentulos or gutta percha tips. Accordingly, the viscosity must be low so that thin films may be formed. Independent from the application technique, the viscosity of the material must be low enough so that the composition may enter into dentine canals in the root canal.

The application of dental root canal sealing compositions is checked by using X-ray procedures. Due to the requirement for radioopacity, the compositions are required to contain a substantial amount of a radioopaque filler.

Dental root canal sealing compositions are known from WO 02/13767 disclosing in the application examples a two-component paste/paste system. The two-component paste/paste system is based on addition polymerisation of equimolar amounts of low-molecular diamines and low-molecular diacrylates optionally in the presence of a reactive diluent for ajusting the viscosity of the composition.

However, the presence of low molecular amines in the dental root canal sealing composition leads to severe drawbacks. Cytotoxic effects are frequently observed due to leaching of such amines from the root canal. Moreover, the cured compositions of WO 02/13767 show a considerable solubility whereby the cytotoxicity problem is aggravated and further application problems are created. Finally, the high vapor pressure of low molecular amines and the high penetration rate through plastic packaging render the compositions of WO 02/13767 problematic for industrial application.

Polyaminoesters specifically disclosed in WO 02/13767 are highly viscous and require the use of a substantial amount of reactive diluent in order to decrease the viscosity. However, reactive diluents cannot be polymerised by addition polymerisation, but require the presence of a polymerisation initiator.

WO 02/13767 does not disclose a di-or polyfunctional acrylate compound or a di- or polyfunctional maleimide compound which is capable of undergoing polyaddition.

It is the problem of the present invention to provide a dental root canal sealing composition having a low viscosity, low cytotoxicity, and low solubility while having excellent mechanical properties such as low shrinkage and flexibility and which do not give rise to handling problems during manufacture and application.

### Summary of the invention

This problem is solved according to the claims. The present invention provides a dental root canal sealing composition curable in the absence of a polymerisation initiator, which comprises
(i) an aminoterminated prepolymer having a viscosity at 23°C of less than 100 Pas, which is obtainable by reacting
   (a) one mole of a compound of the following formula (I) 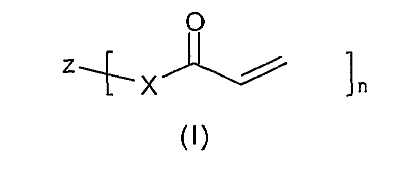 wherein
      X represents a nitrogen or an oxygen atom;
      Z represents an n-valent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
      n represents an integer of from 2 to 6; and
   (b) at least n moles of one or more compounds
      either of the following formula (II) 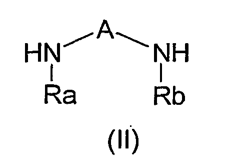 wherein
      - A: represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
      - Rₐ and R_{b}: are the same or different and represent a hydrogen atom, a C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group;
   or of formula (II) in combination with one or more compounds of a formula RNH₂, wherein R represents C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group, and/or in combination with a further di- or polyamine compound;
(ii) a compound capable of undergoing polyaddition with the aminoterminated prepolymer (i);
(iii) 40 to 85 wt.-% of a filler for providing a minimum radioopacity of at least 3mm/mm Al.

The dental root canal sealing composition is in the form of a two-component composition wherein a first component contains the amino terminated prepolymer (i) and optionally filler (iii) and a second component contains the compound (ii) capable of undergoing polyaddition with the aminoterminated prepolymer (i) and optionally filler (iii).

In the formulae, X represents a nitrogen or an oxygen atom.

In the formulae, Z is an n-valent saturated aliphatic C₂₋₁₆ hydrocarbon group or a divalent saturated cycloaliphatic C₃₋₆ hydrocarbon group, which groups may be based on linear or branched alkylene groups having 2 to 16 carbon atoms, preferably 4 to 10 carbon atoms, or cycloalkylene groups having 3 to 6 carbon atoms, preferably 4 to 6 carbon atoms. Z may be divalent (n=2), trivalent (n=3), tetravalent (n=4), pentavalent (n=5), or hexavalent (n=6). Preferable Z is divalent or trivalent. The hydrocarbon group may be substituted by one or more C₁₋₄ alkyl groups. Specific examples of the alkyl groups are methyl, ethyl, n-propyl, i-propyl, n-butyl, i-butyl or tert.-butyl. The hydrocarbon group may contain 1 to 6 oxygen atoms in the carbon chain connecting the n acrylate substituents or in a side chain. Preferably the aliphatic or cycloaliphatic C₂₋₁₆ hydrocarbon group is highly flexible due to the presence of ether bonds and the absence of bulky groups. In a preferred embodiment, Z is a divalent group based on a straight chain alkyl group which may contain ether bonds. Specifically, Z may be a C₂₋₆ alkylene group.

In the formulae, A is a divalent saturated aliphatic C₂₋₁₆ hydrocarbon group or a divalent saturated cycloaliphatic C₃₋₆ hydrocarbon group, which groups may be based on linear or branched alkylene groups having 2 to 16 carbon atoms, preferably 4 to 10 carbon atoms, or cycloalkylene groups having 3 to 6 carbon atoms, preferably 4 to 6 carbon atoms. The hydrocarbon group may be substituted by one or more C₁₋₄ alkyl groups. Specific examples of the alkyl groups are methyl, ethyl, n-propyl, i-propyl, n-butyl, i-butyl or tert.-butyl. The hydrocarbon group may contain 1 to 6 oxygen atoms in the carbon chain connecting the amino groups or in a side chain. Preferably the divalent saturated aliphatic C₂₋₁₆ hydrocarbon group or the divalent saturated cycloaliphatic C₃₋₆ hydrocarbon group is highly flexible due to the presence of ether bonds and the absence of bulky groups. In a preferred embodiment, A is a divalent group based on a straight chain alkylene group which may contain ether bonds. In a preferred embodiment, A may be -(CH₂)₂O(CH₂)₂O(CH₂)₂-.

Rₐ and R_{b} may the same or different and represent a hydrogen atom, a C₁₋₆ alkyl or a C₃₋₄ cycloalkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples of the C₃₋₁₄ cycloalkyl group can include those having 3 to 14 carbon atoms, for example, cyclopropyl, cyclobutyl, cyclopentyl and cyclohexyl. The C₁₋₆ alkyl group and the C₃₋₁₄ cycloalkyl group may optionally be substituted by one or more members of the group selected from a C₁₋₄ alkyl group, C₁₋₄ alkoxy group, a phenyl group, and a hydroxy group. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy. Preferably, Rₐ and R_{b} are hydrogen.

In the preparation of the prepolymer, the compound of formula (II) may be optionally used in combination with an amine compound of the formula RNH₂, wherein R represents C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group, or a further di- or polyamine compound. The amine of the formula RₐNH₂ and/or the further di- or polyamine compound may be used to replace up to n/1.5 moles, preferably n/20 to n/2 moles of the compound of formula (II) used in the reaction for preparing the prepolymer, wherein n is as defined above. The amount of the component used in combination with the diamine of formula (II) must be chosen such that the viscosity of the prepolymer does not exceed 100 Pa*s, preferably 80 Pa*s, more preferably 20 Pa*s.

### Description of the preferred embodiments

In a preferred embodiment the dental root canal sealing composition contains
(i) a prepolymer of the following formula (III) 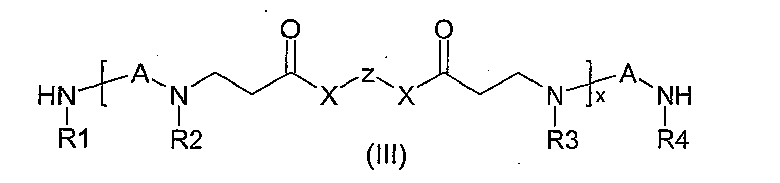 wherein
   - A: represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
   - X: represents an oxygen or nitrogen atom;
   - Z: represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
   - R1, R2, R3, and R4: which may be the same or different, represent a hydrogen atom, a C₁₋₄ alkyl or a C₃₋₁₄ cycloalkyl group, which may be substituted by a C₁₋₄ alkyl group, C₁₋₄ alkoxy group a phenyl group, or a hydroxy group; and
   - x: is an integer of from 1 to 8;
(ii) a compound capable of undergoing polyaddition with the aminoterminated prepolymer (i);
(iii) 40 to 85 wt.-% of a filler for providing a minimum radioopacity of at least 3mm/mm Al.

The dental root canal sealing composition of the invention contains 40 to 85 wt.-% of a filler for providing a minimum radioopacity of the cured composition of at least 3mm/mm Al. The filler contains La₂O₃, ZrO₂, BiPO₄, CaWO₄, BaWO₄, SrF₂, Bi₂O₃. The radioopacity of the cured composition of the invention is at least 3mm/mm Al, preferably at least 5 to 7 mm/mm Al, and most preferably at least 7 mm/mm Al.

Preferably, the dental root canal sealing composition of the invention does not contain a diluent, in particular a reactive diluent, having a viscosity which is lower than the viscosity of the prepolymer of the invention. Moreover, the dental root canal sealing composition does not need to contain a polymerisation initiator. In a preferred embodiment, the dental root canal sealing composition consists essentially of components (i) to (iii). A dental root canal sealing composition consisting essentially of components (i) to (iii) may contain common additives used in the dental field such as colorants, antibiotic agents and ion releasing agents, in a total amount of not more than 25 wt.-%, preferably not more than 10 wt.% of the composition.

A preferred embodiment of the dental root canal sealing composition of the invention contains 40 to 85 wt.-% of a filler and 15 to 60 wt.-% of the aminoterminated prepolymer and the compound capable of undergoing polyaddition with the aminoterminated prepolymer. The aminoterminated prepolymer used in the present invention is usually a mixture of oligomers. Accordingly, the amount of the aminoterminated prepolymer and the compound capable of undergoing polyaddition with the aminoterminated prepolymer is calculated based on the mixture of oligomers.

The dental root canal sealing composition of the present invention is a two component composition which is mixed prior to use.The two component composition is preferably a powder/liquid system, a powder/paste system, a paste/paste system or a liquid/paste system. The paste/paste system or a liquid/paste system may be applied by an applicator wherein both components are mixed by a static mixer.

The present invention is based on the recognition that the Michael addition of specific diamines to specific di- and oligoacrylate compounds provides prepolymers having low viscosity while at the same time eliminating the problems associated with the presence of low molecular amines. Surprisingly, the reaction kinetics of the Michael addition of diamines with acrylates differs from the reaction kinetics of other addition reactions of amines so that the reaction products are not crosslinked or high molecular highly viscous materials, but uncrosslinked prepolymers having low viscosity. It appears that the difference of the reaction rates between primary and secondary amines with acrylate compounds is the basis for the possibility of obtaining the compositions of the present invention. Based on the specific reaction kinetics of primary amines in the present invention, the content of residual primary amines in the prepolymer or in the dental root canal sealing composition of the present invention is substantially reduced as compared to systems using functional end groups showing the same reactivity towards primary and secondary amines, cf. J. Klee, H.-H. Hörhold, J. Raddatz; Acta Polymerica, 41 (1990) 557-560; "Telechele Prepolymere aus DGEBA und disekundären Diaminen. Unvernetzte Epoxid-Amin-Additionspolymere, 29."; J.E. Klee; Acta Polym. 45 (1994) 73-82; "Telechelic prepolymers and macromonomers by step growth processes" (39); J. E. Klee, R.-E. Grützner, H.-H. Hörhold; Macromol. Chem. Phys. 197 (1996) 2305-2323; Linear aryalamine/ Bis-2,2-[4-(2,3-epoxypropoxy)-phenyl]-propane addition polymers - synthesis and properteis, Uncrosslinked epoxide-amine addition polymers, 44." Primary amines react much faster as compared to secondary amines, in particular in the reaction with di- or polyfunctional acrylates or acrylamides, or bis- or polymaleimides.

The prepolymer contained in the dental root canal sealing composition of the present invention has a viscosity at 23°C of less than 100 Pa*s. Preferably, the viscosity of the prepolymer is in the range of from 1 to 80 Pa*s, more preferably from 1 to 20 Pa*s. If the viscosity is too high, then it will be difficult to apply the composition through the canal of a needle. If the viscosity is too low, then it will be difficult handle the composition.

The dental root canal sealing composition of the present invention is curable in the absence of a polymerisation initiator. The curing mechanism is based on an addition reaction of an addition reaction between the aminoterminated prepolymer (i) and a compound capable of undergoing polyaddition with the aminoterminated prepolymer (i). The compound capable of undergoing polyaddition with the aminoterminated prepolymer (i) is a di- or polyfunctional acylate, or di- or polyfunctional maleimide. Di-or polyfunctional acrylates and di- or polyfunctional maleimides are preferred with regard to the selectivity in the reaction with primary and secondary amino groups. The compound capable of undergoing polyaddition with the aminoterminated prepolymer (i) may be the same or different as the compound as defined by formula (I) wherein X and Z are as defined above and n represents an integer of from 2 to 6.

The use of the specific amino terminated prepolymers eliminates or at least strongly reduces the problems associated with low molecular amines. Moreover, it is surprising that the use of the specific prepolymers provides a dental root canal sealing composition which has a low viscosity. For this purpose, it is essential that rigid moieties in the prepolymers are avoided.

The compositions of the pesent invention may be applied to a root canal by using conventional techniques. Specifically, the compositions of the present invention may be applied via the canal of a syringe into the root canal. Moreover, the compositions of the present invention may also be used for the manufacture of prefabricated root canal cones. If cones made of the compositions of the invention are used in combination with the respective dental root canal sealing composition of the invention, compatibility of the cones with the sealing composition can be guaranteed whereby a tight seal may be obtained. The cured product obtained with the composition according to the invention has superior mechanical properties, in particular with regard to flexibility, which is essential for the application as a root canal sealing composition.

Now, the general process for the preparation of the an amino terminated prepolymer will be disclosed. The prepolymer is obtainable by reacting
(a) one mole of a compound of the following formula (I) 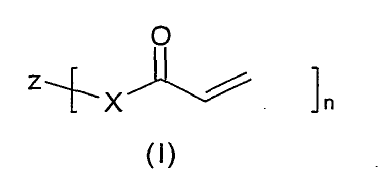 wherein
   X and Z are as defined above and
   n represents an integer of from 2 to 6; and
(b) at least n moles of one or more compounds of the following formula (II) 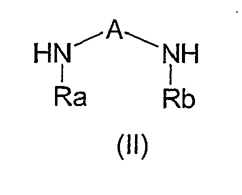
wherein A, Rₐ, and R_{b} are as defined above.

The reaction may be carried out in the absence of a solvent or in the presence of a suitable solvent. The temperature of the reaction is preferably in the range of from 10 °C to 150 °C, more preferably in the range of 20 °C to 80 °C. The reaction time depends on the temperature and the reactivity of the reaction system and is usually in the range of from hours to several days. The termination of the reaction may be checked by conventional methods sucha s an IR spectrum whereby the end of the reaction is reached when all acrylic carbon-carbon double bonds have disappeared. In case the reaction is carried out in the absence of a solvent, the prepolymers obtained by the reaction of compounds (I) and (II) may be used as such without further work-up of the reaction mixture. In the preparation of the prepolymer, the compound of formula (II) may be used in combination with an amine of the formula RₐNH₂ wherein Rₐ is as defined above. The amine of the formula RₐNH₂ may be used to replace of from n/10 to n/2 moles of the compound of formula (II) used in the reaction for preparing the prepolymer.

The present invention will now be further explained with reference to specific examples. Dynamic viscosities were measured by using a Bohlin CS50 rheometer at 23°C.

### EXAMPLES

### Example 1

In a 250 ml flask equipped with stirrer and condenser 22.955 g (154.88 mmol) 2,2-(Ethylendioxy)-diethylene amine, 15.350 g (77.44 mmol) butanediol diacrylate were homogeneously mixed and stirred at 20 to 25 °C for 3 days. Thereafter no double bonds were found in the IR-spectrum at 1609 and 809 cm⁻¹. Yield: 37.764 g (100 % d. Th), η 23 °C = 5.865 ± 0.148 Pa*s (after storage for 1 month at room temperature) 2.036 g (4.197 mmmol) of the prepared prepolymer were homogeneously mixed with 2.149 g (4.197 mmol) ethoxylated bisphenol A diacrylate (SR-601, Sartomer) and reacted at 37 °C. The mixture has a gel time of 3 hours at 37 °C.

### Example 2

In a 250 ml flask equipped with stirrer and condenser 15.000 g (77.19 mmol) 3,(4),8,(9)-bis(aminomethyl)-tricyclo-5.2.1.0 ^{2,6}-decane, 11.441 g (77.19 mmol) 2,2-(ethylendioxy)-diethylene amine and 15.301 g (77.19 mmol) butanediol diacrylate were homogeneously mixed and stirred for 3 days at room temperature. Thereafter no double bonds were found in the IR-spectrum at 1609 and 809 cm⁻¹.
Yield: 41.742 g (100 % d. Th),η 23 °C = 9.815 ± 0.174 Pa*s

2.586 g (4.782 mmol) of the prepared prepolymer were homogeneously mixed with 2.449 g (4.782 mmol) ethoxylated bisphenol A diacrylate (SR-601, Sartomer) and reacted at 37 °C. The mixture has a gel time of 6 to 16 hours at 37 °C and becomes solid.

### Application Example 1 (Powder/Liquid)

1.000 g of amino terminated prepolymer prepared by addition reaction of 2,2-(ethylenedioxy)diethylene amine and butanediol diacrylate of example 1 and 3.050 g of a powder composed of cyclohexane dimethanol diacrylate (12.594 wt.-%), calcium tungstate (50.174 wt.-%), and zirconium oxide (12.544 wt.-%) were homogenously mixed and polymerised for 3 hours.

### Application Example 2 (Paste/Paste)

- Acrylate Paste:: 5.000 g (9.77 mmol) ethoxylated bisphenol A diacrylate (SR-601, Sartomer). 9.917 g calcium tungstate, 2.479 g zirconium oxide, 0.050 g arosil A 200 and 0.025 g iron (III) oxide were homogeneously mixed.
- Prepolymer Paste:: 4.830 g (9.77 mmol) aminoterminated prepolymer according example 6, 9.500 g calcium tungstate, 2.375 g zirconium oxide and 0.536 g arosil A 200 were homogeneously mixed.

Immediately before application 1.00 g of the acrylate paste and 0.987 g of the Amino-Prepolymer Paste were mixed homogeneously and polymerised at 37 °C for 3 hours.

### Reference Example

In a 250 ml flask equipped with stirrer and condenser 25.000 g (128.70 mmol) 3,(4),8,(9)-bis(aminomethyl) tricydo-5.2.1.0 ^{2,6} decane, 12.755 g (64.35 mmol) butanediol diacrylate were homogeneously mixed and stirred at 20 to 25 °C for 3 days. Thereafter no double bonds were found in the IR-spectrum at 1609 and 809 cm⁻¹. Yield: 37.764 g (100 % d. Th), η 23°C = 7157 ± 100 Pa*s (after storage for 1 month at room temperature)

2.127 g (3.624 mmol) of the prepolymer were homogeneously mixed with 0.716 g (2.416 mmol) trimethylol propane triacrylate and reacted at 37 °C. The mixture has a gel time of 30 minutes at 37 °C.

The prepolymer has a viscosity which is not suitable for the application as a root canal sealing composition. This example shows that the reaction product of two moles of a diamine disclosed in application example 1 of WO02/13767 and one mole of a diacrylate provides a prepolymer having excessive viscosity. In case of the reaction product of equimolar amounts of a diamine and a diacrylate as disclosed in application example 1 of WO02/13767, the viscosity of the polymeric product will even be higher.

## Claims

1. A dental root canal sealing composition curable in the absence of a polymerisation initiator, which comprises
(i) an amino terminated prepolymer having a viscosity at 23°C of less than 100 Pas, which is obtainable by reacting
(a) one mole of a compound of the following formula (I) 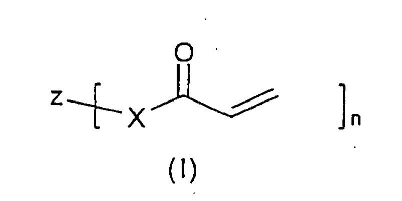 wherein
X represents a nitrogen or an oxygen atom;
Z represents an n-valent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
n represents an integer of from 2 to 6;
(b) at least n moles of one or more compounds
either of the following formula (II) 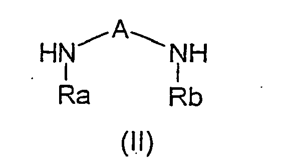 wherein
A represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
Rₐ and R_{b} are the same or different and represent a hydrogen atom, a C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group;
or of formula (II) in combination with one or more compounds of a formula RNH₂, wherein R represents C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group, and/or in combination with a further di- or polyamine compound;
(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm Al;
said compositon being in the form of a two-component composition wherein a first component contains the amino terminated prepolymer (i) and optionally filler (iii) and a second component contains the compound (ii) capable of undergoing polyaddition with the aminoterminated prepolymer (i) and optionally filler (iii).

2. The dental root canal sealing composition according to claim 1 wherein the composition contains a prepolymer of the following formula (III) 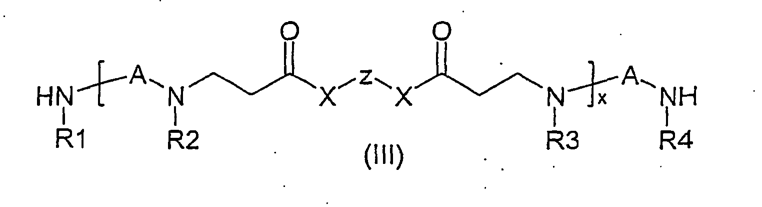
A represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
X represents an oxygen or nitrogen atom;
Z represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
R1, R2, R3, and R4 which may be the same or different, represent a hydrogen atom, a C₁₋₄ alkyl or a C₃₋₁₄ cycloalkyl group, which may be substituted by a C₁₋₄ alkyl group, C₁₋₄ alkoxy group a phenyl group, or a hydroxy group; and
x is an integer of from 1 to 8;
(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm Al.

3. The dental root canal sealing composition according to claims 2, wherein
A represents a divalent saturated aliphatic C₂₋₁₀ hydrocarbon chain which may contain 2 to 4 oxygen atoms, and which may be substituted by 1 to 6 C₁₋₄ alkyl groups.

4. The dental root canal sealing composition according to claim 1, wherein
A represents -(CH₂)₂O(CH₂)₂O(CH₂)₂-.

5. The dental root canal sealing composition according to any one of the preceding claims, wherein
Z represents a divalent saturated aliphatic C₂₋₁₀ hydrocarbon chain, which may be substituted by 1 to 6 C₁₋₄ alkyl groups.

6. The dental root canal sealing composition according to any one of the preceding claims, wherein Z represents a C₂₋₆ alkylene group.

7. The dental root canal sealing composition according to any one of the preceding claims, wherein X represents an oxygen atom.

8. The dental root canal sealing composition according to any one of the preceding claims, wherein X represents a nitrogen atom.

9. The dental root canal sealing composition according to any one of the preceding claims, wherein R1, R2, R3, and R4 are the same, and preferably a hydrogen atom.

10. The dental root canal sealing composition according to any one of the preceding claims, wherein R1, R2, R3, and R4 are selected from a hydrogen atom, a C₁₋₄ alkyl group, which may be substituted by a phenyl group, a hydroxy group, or an amino group.

11. The dental root canal sealing composition according to any one of the preceding claims; wherein the filler contains La₂O₃, ZrO₂, BiPO₄, CaWO₄, BaWO₄, SrF₂, Bi₂O_{3.}

12. The dental root canal sealing composition according to any one of the preceding claims, wherein the two-component composition is a powder/liquid or a paste/paste system.

13. The dental root canal sealing composition according to any one of the preceding claims, containing a prepolymer of the following formula: 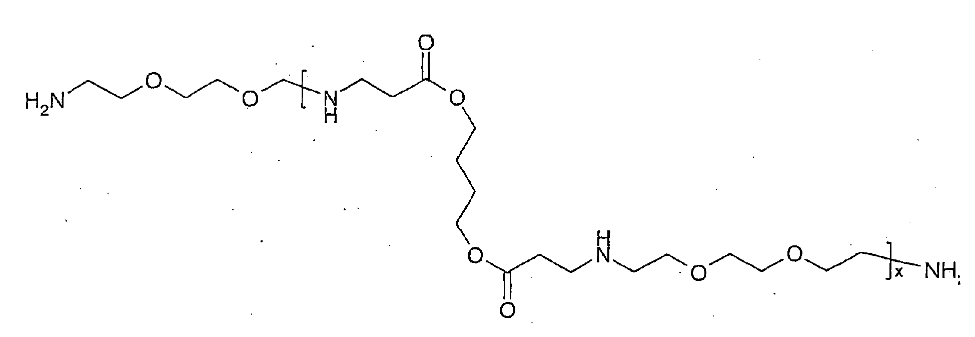 wherein x is as defined above.

14. The dental root canal sealing composition according to any one of the preceding claims, wherein the amino terminated prepolymer is obtainable by reacting
(i) one mole of a compound of the following formula (IV) 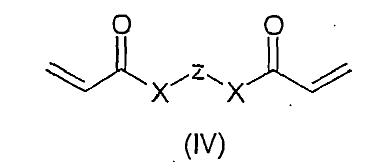 wherein X and Z are as defined as in claim 2,
(ii) at least two moles of one or more compounds of the following formula (III) 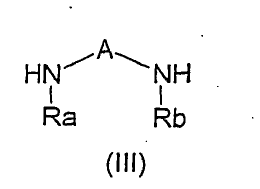 wherein A is as defined in claim 2 and Ra and Rb are the same or different and represent a hydrogen atom, a C₁₋₄ alkyl or a C₃₋₁₄ cycloalkyl group, which may be substituted by a C₁₋₄ alkyl group, a phenyl group, or a hydroxy group, and
(iii) optionally an amine compound of the formula RNH₂, wherein
R represents C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group, or a further di- or polyamine compound.

## Patentansprüche

1. Zusammensetzung zur Abdichtung von Zahnwurzelkanälen, die in Abwesenheit eines Polymerisationsinitiators härtbar ist, die aufweist
(i) ein Amino-terminiertes Prepolymer, das eine Viskosität bei 23°C von weniger als 100 Pas aufweist, und erhältlich ist durch Umsetzen von
(a) einem Mol einer Verbindung der folgenden Formel (I) 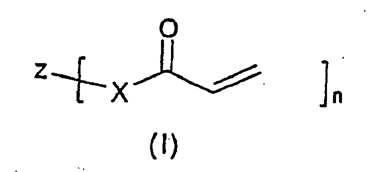 worin bedeuten
X ein Stickstoff- oder ein Sauerstoffatom;
Z eine n-wertige gesättigte aliphatische C₂₋₁₆-Kohlenwasserstoffgruppe oder eine zweiwertige gesättigte cycloaliphatische C₃₋₆-Kohlenwasserstoffgruppe, wobei die Gruppen 1 bis 6 Sauerstoffatome enthalten können, und die durch eine bis sechs C₁₋₄-Alkylgruppen substituiert sein können; und
n eine ganze Zahl von 2 bis 6 bedeutet;
(b) mindestens n Mol einer oder mehrerer Verbindungen der folgenden Formel (II) 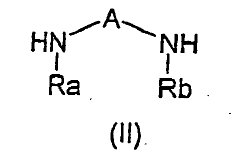
worin
A eine zweiwertige gesättigte aliphatische C₂₋₁₆-Kohlenwasserstoffgruppe oder eine zweiwertige gesättigte cycloaliphatische C₃₋₆-Kohlenwasserstoffgruppe bedeutet, wobei die Gruppen 1 bis 6 Sauerstoffatome enthalten können, und durch eine bis sechs C₁₋₄-Alkylgruppen substituiert sein können;
Rₐ und R_{b} gleich oder verschieden sind und ein Wasserstoffatom, eine C₁₋₆-Alkyl- oder eine C₃₋₁₄-Cycloalkylgruppe bedeuten, die substituiert sein kann durch einen oder mehrere Reste der Gruppe, ausgewählt aus C₁₋₄-Alkylgruppe, C₁₋₄-Alkoxygruppe, Phenylgruppe und Hydroxygruppe;
oder von Formel (II) in Kombination mit einer oder mehreren Verbindungen der Formel RNH₂, worin R eine C₁₋₆-Alkyl- oder eine C₃₋₁₄-Cycloalkylgruppe bedeutet, die substituiert sein kann durch ein oder mehrere Reste der Gruppe, ausgewählt aus einer C₁₋₄-Alkylgruppe, C₁₋₄-Alkoxygruppe, Phenylgruppe und Hydroxygruppe, und/oder in Kombination mit einer weiteren Di- oder Polyaminverbindung;
(ii) eine di- oder polyfunktionelle Acrylatverbindung oder eine di- oder polyfunktionelle Maleimidverbindung, die zu einer Polyaddition mit dem Amino-terminierten Prepolymer (i) fähig ist;
(iii) 40 bis 85 Gew.-% eines Füllstoffs zur Bildung einer minimalen Radioopazität von mindestens 3 mm/mm Al;
wobei die Zusammensetzung in Form einer Zweikomponenten-Zusammensetzung vorliegt, worin eine erste Komponente das Amino-terminierte Prepolymer (i) und gegebenenfalls den Füllstoff (iii) enthält, und eine zweite Komponente die Verbindung (ii), die zur Polyaddition mit dem Amino-terminierten Prepolymer (i) fähig ist, und gegebenenfalls Füllstoff (iii) enthält.

2. Zusammensetzung zur Abdichtung von Zahnwurzelkanälen nach Anspruch 1, worin die Zusammensetzung ein Prepolymer der folgenden Formel (III) enthält 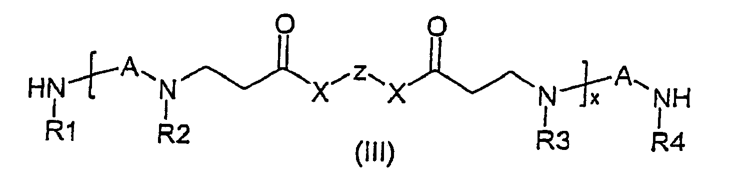 worin
A eine zweiwertige gesättigte aliphatische C₂-₁₆-Kohlenwasserstoffgruppe oder eine zweiwertige gesättigte cycloaliphatische C₃₋₆-Kohlenwasserstoffgruppe bedeutet, wobei die Gruppen 1 bis 6 Sauerstoffatome enthalten können, und durch eine bis sechs C₁₋₄-Alkylgruppen substituiert sein können;
X ein Sauerstoff- oder Stickstoffatom bedeutet;
Z eine zweiwertige gesättigte aliphatische C₂₋₁₆-Kohlenwasserstoffgruppe oder eine zweiwertige gesättigte cycloaliphatische C₃₋₆-Kohlenwasserstoffgruppe bedeutet, wobei die Gruppen 1 bis 6 Sauerstoffatome enthalten können und durch eine bis sechs C₁₋₄-Alkylgruppen substituiert sein können; und
R1, R2, R3 und R4, die gleich oder verschieden sein können, ein Wasserstoffatom, eine C₁₋₆-Alkyl- oder eine C₃-₁₄-Cycloalkylgruppe bedeuten, die durch eine C₁₋₄-Alkylgruppe, C₁₋₄-Alkoxygruppe, eine Phenylgruppe oder eine Hydroxygruppe substituiert sein kann; und
x eine ganze Zahl von 1 bis 8 bedeutet;
(ii) eine di- oder polyfunktionelle Acrylatverbindung oder eine di- oder polyfunktionelle Maleimidverbindung, die zu einer Polyaddition mit dem Amino-terminierten Prepolymer (i) fähig ist;
(iii) 40 bis 85 Gew.-% eines Füllstoffs zur Bildung einer minimalen Radioopazität von mindestens 3 mm/mm Al.

3. Zusammensetzung zur Abdichtung von Zahnwurzelkanälen nach Anspruch 2, worin
A eine zweiwertige gesättigte aliphatische C₂₋₁₀-Kohlenwasserstoffkette bedeutet, die 2 bis 4 Sauerstoffatome enthalten kann und durch eine bis sechs C₁₋₄-Alkylgruppen substituiert sein kann.

4. Zusammensetzung zur Abdichtung von Zahnwurzelkanälen nach Anspruch 1, worin
A -(CH₂)₂O(CH₂)₂O(CH₂)₂- bedeutet.

5. Zusammensetzung zur Abdichtung von Zahnwurzelkanälen nach einem der vorhergehenden Ansprüche, worin
Z eine zweiwertige gesättigte aliphatische C₂₋₁₀-Kohlenwasserstoffkette bedeutet, die durch eine bis sechs C₁₋₄-Alkylgruppen substituiert sein kann.

6. Zusammensetzung zur Abdichtung von Zahnwurzelkanälen nach einem der vorhergehenden Ansprüche, worin Z eine C₂₋₆-Alkylengruppe bedeutet.

7. Zusammensetzung zur Abdichtung von Zahnwurzelkanälen nach einem der vorhergehenden Ansprüche, worin X eine Sauerstoffatom bedeutet.

8. Zusammensetzung zur Abdichtung von Zahnwurzelkanälen nach einem der vorhergehenden Ansprüche, worin X ein Stickstoffatom bedeutet.

9. Zusammensetzung zur Abdichtung von Zahnwurzelkanälen nach einem der vorhergehenden Ansprüche, worin R1 R2, R3 und R4 gleich sind und vorzugsweise ein Wasserstoffatom bedeuten.

10. Zusammensetzung zur Abdichtung von Zahnwurzelkanälen nach einem der vorhergehenden Ansprüche, worin R1, R2, R3 und R4 ausgewählt sind aus einem Wasserstoffatom, einer C₁₋₄-Alkylgruppe, die durch eine Phenylgruppe, eine Hydroxygruppe oder eine Aminogruppe substituiert sein kann.

11. Zusammensetzung zur Abdichtung von Zahnwurzelkanälen nach einem der vorhergehenden Ansprüche, worin der Füllstoff La₂O₃, ZrO₂, BiPO₄, CaWO₄, BaWO₄, SrF₂, Bi₂O₃ enthält.

12. Zusammensetzung zur Abdichtung von Zahnwurzelkanälen nach einem der vorhergehenden Ansprüche, worin die Zweikomponenten-Zusammensetzung ein Pulver/Flüssigkeit- oder ein Pasten/Pasten-System ist.

13. Zusammensetzung zur Abdichtung von Zahnwurzelkanälen nach einem der vorhergehenden Ansprüche, das ein Prepolymer der folgenden Formel enthält: 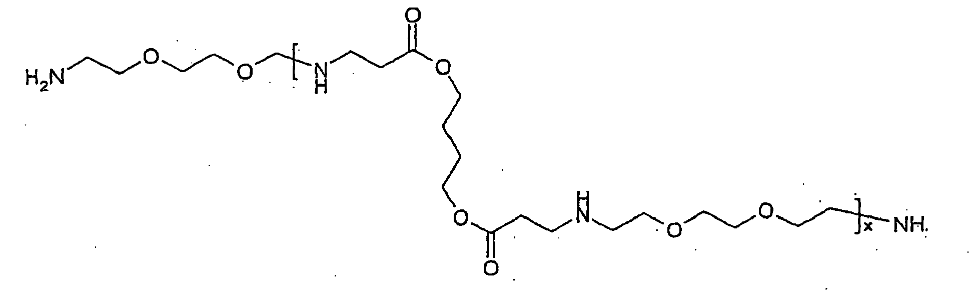 worin x die vorstehend angegebene Bedeutung besitzt.

14. Zusammensetzung zur Abdichtung von Zahnwurzelkanälen nach einem der vorhergehenden Ansprüche, worin das Amino-terminierte Prepolymer erhältlich ist durch Umsetzen von
(i) einem Mol einer Verbindung der folgenden Formel (IV) 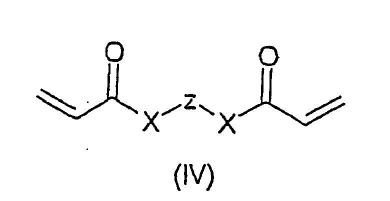 worin X und Z die in Anspruch 2 angegebene Bedeutung besitzen,
(ii) mindestens zwei Mol einer oder mehrerer Verbindungen der folgenden Formel (III) 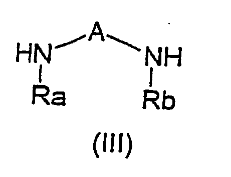 worin A die in Anspruch 2 angegebene Bedeutung besitzt, und Ra und Rb gleich oder verschieden sein können und ein Wasserstoffatom, eine C₁₋₄-Alkyl- oder eine C₃₋₁₄-Cycloalkylgruppe bedeuten, die durch eine C₁₋₄-Alkylgruppe, eine Phenylgruppe oder eine Hydroxygruppe substituiert sein kann, und
(iii) gegebenenfalls einer Aminverbindung der Formel RNH₂, worin
R C₁₋₆-Alkyl oder eine C₃₋₁₄-Cycloalkylgruppe bedeutet, die substituiert sein kann durch ein oder mehrere Reste der Gruppe, ausgewählt aus einer C₁₋₄-Alkylgruppe, C₁₋₄-Alkoxygruppe, einer Phenylgruppe und einer Hydroxygruppe, oder einer weiteren Di- oder Polyaminverbindung.

## Revendications

1. Composition d'obturation de canal radiculaire dentaire, durcissable en l'absence d'inhibiteur de polymérisation, qui comprend
(i) un prépolymère à terminaison amino ayant une viscosité à 23°C inférieure à 100 Pas, qui peut être obtenu en faisant réagir
(a) une mole d'un composé de formule (I) suivante 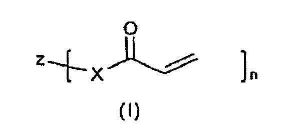 dans laquelle
X représente un atome d'azote ou un atome d'oxygène ;
Z représente un groupe hydrocarboné en C₂ à C₁₆ aliphatique saturé de valence n ou un groupe hydrocarboné en C₃ à C₆ cycloaliphatique saturé divalent, ces groupes pouvant contenir 1 à 6 atomes d'oxygène et pouvant être substitués avec 1 à 6 groupes alkyle en C₁ à C₄ ; et
n représente un nombre entier de 2 à 6 ;
(b) au moins n moles d'un ou plusieurs composés de formule (II) suivante 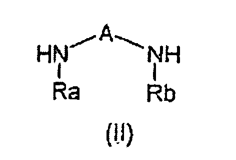
dans laquelle
A représente un groupe hydrocarboné en C₂ à C₁₆ aliphatique saturé divalent ou un groupe hydrocarboné en C₃ à C₆ cycloaliphatique saturé divalent, ces groupes pouvant contenir 1 à 6 atomes d'oxygène et pouvant être substitués avec 1 à 6 groupes alkyle en C₁ à C₄ ;
Ra et Rb sont identiques ou différents et représentent un atome d'hydrogène, un groupe alkyle en C₁ à C₆ ou un groupe cycloalkyle en C₃ à C₁₄, qui peut être substitué avec un ou plusieurs membres du groupe choisi entre un groupe alkyle en C₁ à C₄, un groupe alkoxy en C₁ à C₄, un groupe phényle et un groupe hydroxy ;
ou bien la formule (II) en association avec un ou plusieurs composés de formule RNH₂, dans laquelle R représente un groupe alkyle en C₁ à C₆ ou un groupe cycloalkyle en C₃ à C₁₄, qui peut être substitué avec un ou plusieurs membres du groupe choisi entre un groupe alkyle en C₁ à C₄, un groupe alkoxy en C₁ à C₄, un groupe phényle et un groupe hydroxy, et/ou en association avec une di- ou polyamine supplémentaire ;
(ii) un acrylate di- ou polyfonctionnel ou un maléimide di- ou polyfonctionnel qui est capable de subir une polyaddition avec le prépolymère à terminaison amino (i) ;
(iii) 40 à 85 % en poids d'une charge pour parvenir à une radio-opacité minimale d'au moins 3 mm/mm de Al ;
ladite composition étant sous forme d'une composition à deux constituants dans laquelle un premier constituant contient le prépolymère à terminaison amino (i) et facultativement une charge (iii), et un second constituant contient le composé (ii) capable de subir une polyaddition avec le prépolymère à terminaison amino (i) et la charge facultative (iii).

2. Composition d'obturation de canal radiculaire dentaire suivant la revendication 1, qui contient
(i) un prépolymère de formule (III) suivante 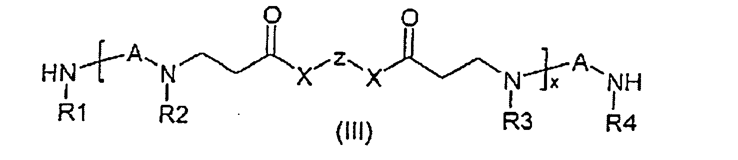 dans laquelle
A représente un groupe hydrocarboné en C₂ à C₁₆ aliphatique saturé divalent ou un groupe hydrocarboné en C₃ à C₆ cycloaliphatique saturé divalent, ces groupes pouvant contenir 1 à 6 atomes d'oxygène et pouvant être substitués avec 1 à 6 groupes alkyle en C₁ à C₄ ;
X représente un atome d'oxygène ou d'azote ;
Z représente un groupe hydrocarboné en C₂ à C₁₆ aliphatique saturé divalent ou un groupe hydrocarboné en C₃ à C₆ cycloaliphatique saturé divalent, ces groupes pouvant contenir 1 à 6 atomes d'oxygène et pouvant être substitués avec 1 à 6 groupes alkyle en C₁ à C₄ ; et
R1, R2, R3 et R4, qui peuvent être identiques ou différents, représentent un atome d'hydrogène, un groupe alkyle en C₁ à C₄ ou un groupe cycloalkyle en C₃ à C₁₄, qui peut être substitué avec un groupe alkyle en C₁ à C₄, un groupe alkoxy en C₁ à C₄, un groupe phényle ou un groupe hydroxy ; et
X représente un nombre entier de 1 à 8 ;
(ii) un acrylate di- ou polyfonctionnel ou un maléimide di- ou polyfonctionnel qui est capable de subir une polyaddition avec le prépolymère à terminaison amino (i) ;
(iii) 40 à 85 % en poids d'une charge pour parvenir à une radio-opacité minimale d'au moins 3 mm/mm de Al.

3. Composition d'obturation de canal radiculaire dentaire suivant la revendication 2, dans laquelle A représente une chaîne hydrocarbonée en C₂ à C₁₀ aliphatique saturée divalente qui peut contenir 2 à 4 atomes d'oxygène et qui peut être substituée avec 1 à 6 groupes alkyle en C₁ à C₄.

4. Composition d'obturation de canal radiculaire dentaire suivant la revendication 1, dans laquelle
A représente un groupe -(CH₂)₂O(CH₂)₂O(CH₂)₂-.

5. Composition d'obturation de canal radiculaire dentaire suivant l'une quelconque des revendications précédentes, dans laquelle
Z représente une chaîne hydrocarbonée en C₂ à C₁₀ aliphatique saturée divalente, qui peut être substituée avec 1 à 6 groupes alkyle en C₁ à C₄.

6. Composition d'obturation de canal radiculaire dentaire suivant l'une quelconque des revendications précédentes, dans laquelle Z représente un groupe alkylène en C₂ à C₆.

7. Composition d'obturation de canal radiculaire dentaire suivant l'une quelconque des revendications précédentes, dans laquelle X représente un atome d'oxygène.

8. Composition d'obturation de canal radiculaire dentaire suivant l'une quelconque des revendications précédentes, dans laquelle X représente un atome d'azote.

9. Composition d'obturation de canal radiculaire dentaire suivant l'une quelconque des revendications précédentes, dans laquelle R1, R2, R3 et R4 sont identiques et représentent de préférence un atome d'hydrogène.

10. Composition d'obturation de canal radiculaire dentaire suivant l'une quelconque des revendications précédentes, dans laquelle R1, R2, R3 et R4 sont choisis entre un atome d'hydrogène, un groupe alkyle en C₁ à C₄, qui peut être substitué avec un groupe phényle, un groupe hydroxy ou un groupe amino.

11. Composition d'obturation de canal radiculaire dentaire suivant l'une quelconque des revendications précédentes, dans laquelle la charge contient La₂O₃, ZrO₂, BiPO₄, CaWO₄, BaWO₄, SrF₂, Bi2O₃.

12. Composition d'obturation de canal radiculaire dentaire suivant l'une quelconque des revendications précédentes, la composition à deux constituants étant un système poudre/liquide ou un système pâte/pâte.

13. Composition d'obturation de canal radiculaire dentaire suivant l'une quelconque des revendications précédentes, contenant un prépolymère de formule suivante : 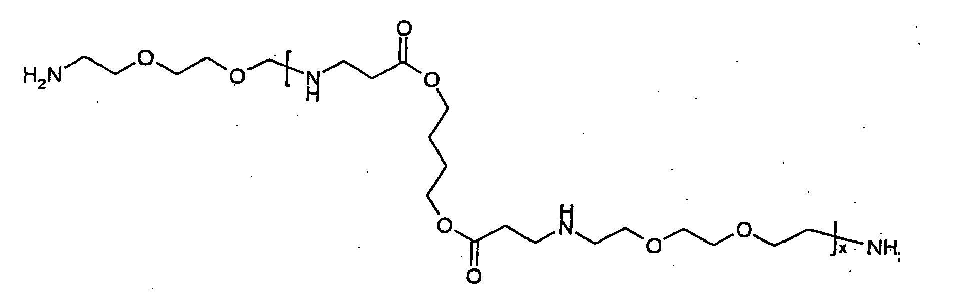 dans laquelle X répond à la définition précitée.

14. Composition d'obturation de canal radiculaire dentaire suivant l'une quelconque des revendications précédentes, dans laquelle le prépolymère à terminaison amino peut être obtenu en faisant réagir
(i) une mole d'un composé de formule (IV) suivante 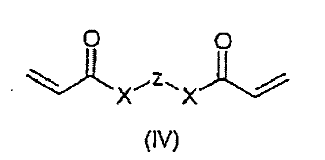 dans laquelle X et Z sont tels que définis dans la revendication 2,
(ii) au moins deux moles d'un ou plusieurs composés de formule (III) suivante 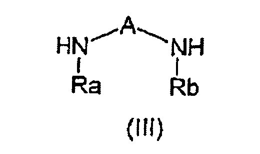 dans laquelle A est tel que défini dans la revendication 2 et Ra et Rb sont identiques ou différents et représentent un atome d'hydrogène, un groupe alkyle en C₁ à C₉ ou un groupe cycloalkyle en C₃ à C₁₄, qui peut être substitué avec un groupe alkyle en C₁ à C₉, un groupe phényle, ou un groupe hydroxy, et
(iii) facultativement, une amine de formule RNH₂,
dans laquelle R représente un groupe alkyle en C₁ à C₆ ou un groupe cycloalkyle en C₃ à C₁₄, qui peut être substitué avec un ou plusieurs membres du groupe choisi entre un groupe alkyle en C₁ à C₄, un groupe alkoxy en C₁ à C₄, un groupe phényle, un groupe hydroxy, ou une di- ou polyamine supplémentaire.
